# FASCICULE DE BREVET EUROPEEN

(11) **EP 3 692 543 B1**
(45) Date de publication et mention de la délivrance du brevet: **07.06.2023**
(21) Numéro de dépôt: 18782106.1
(22) Date de dépôt: 10.09.2018
(51) Int. Cl.: G16H 20/30, A63B 23/00

(54) **DISPOSITIF POUR LA MISE EN OEUVRE DE LA THERAPIE MIROIR**
VORRICHTUNG ZUR DURCHFÜHRUNG EINER SPIEGELTHERAPIE
DEVICE FOR CARRYING OUT MIRROR THERAPY

(30) Priorité: 04.10.2017 FR 1759282
(43) Date de publication de la demande: 12.08.2020
(73) Titulaire: Dessintey, 42650 St Jean Bonnefonds (FR)
(72) Inventeur: FOURNIER, Nicolas, 42230 St Victor Sur Loire (FR); LUNEAU, Davy Christophe, 42400 St Chamond (FR)
(74) Mandataire: Cabinet Laurent & Charras
(86) Numéro de dépôt international: PCT/FR2018/052209
(87) Numéro de publication internationale: WO 2019/068978

(56) Documents cités:
- CN-U- 204 016 761
- US-A1- 2008 306 572
- US-A1- 2015 313 793
- HSIN-MIN LEE ET AL: "Delayed mirror visual feedback presented using a novel mirror therapy system enhances cortical activation in healthy adults", JOURNAL OF NEUROENGINEERING AND REHABILITATION, BIOMED CENTRAL, LONDON, GB, vol. 12, no. 1, 11 juillet 2015 (2015-07-11), page 56, XP021227785, ISSN: 1743-0003, DOI: 10.1186/S12984-015-0053-1

## Description

### DOMAINE TECHNIQUE

La présente invention concerne le domaine technique des équipements de réhabilitation et rééducation neuromotrice.

### ART ANTERIEUR

Il est connu de l'état de la technique des appareils de réhabilitation et rééducation neuromotrice pour mettre en oeuvre une thérapie miroir. Elle consiste à faire croire au cerveau qu'un membre déficient est sain par un système d'illusion d'optique.

Classiquement, un miroir peut être utilisé de sorte que lorsqu'un patient regarde une réflexion d'un membre sain dans ce miroir, le patient ait l'impression de mouvoir un membre déficient. Ainsi, grâce à la plasticité du cerveau, la remodélisation de connexions neurales est possible, avec des résultats avantageux pour remédier aux douleurs chroniques, ou à une déficience motrice du membre. Un exemple de dispositif pour la thérapie miroir est décrit dans le brevet GB 2 436 150.

Plus récemment, des appareils électroniques ont été développés pour la thérapie miroir. Ces appareils comprennent une caméra, un écran et un système de support, pour assurer une bonne position relative entre les différents éléments de l'appareil et le patient. Ainsi, la réflexion d'un membre est remplacée par une image modifiée numériquement. De tels dispositifs sont divulgués par exemple dans les demandes RO 130153, US 2015 313793 et CN 204016761.

Un appareil électronique présente généralement l'inconvénient de nécessiter un temps de réglage important avant utilisation. En effet, l'appareil doit être réglé en fonction de la morphologie de chaque patient, notamment selon sa carrure, son maintien, sa posture et ses possibilités de mouvement, influencés par ses déficiences ou son handicap. Du fait du temps important d'installation du patient à chaque séance, l'efficacité du traitement s'en trouve diminué.

De plus, la qualité de l'illusion est souvent médiocre : elle ne reproduit pas la vision oeil main à l'identique de ce que verrait le patient sans le dispositif. L'efficacité de l'illusion s'en trouve altérée.

### EXPOSE DE L'INVENTION

L'un des buts de l'invention est donc de proposer un appareil qui sera simple à mettre en oeuvre, avec une capacité de réglage facile. Il est à noter que la zone de travail doit être toujours bien choisie pour générer une image plausible, qui est indispensable pour obtenir un effet d'illusion constituant le fondement de la thérapie.

A titre d'exemple, une personne hémiplégique subit souvent des rétractions musculaires de ses membres supérieurs, et a donc des difficultés à positionner son bras sur le plateau de travail. Il existe ainsi un besoin d'avancer l'écran à lui, tout en filmant la bonne zone de travail.

En pratique, la technique consiste à filmer le membre sain qui ne subit pas de rétractions. Il faut pouvoir filmer correctement le bras sain du patient posé sur le plateau, puis inverser la vidéo, et diffuser cette vidéo par-dessus le bras pathologique du patient qui a des rétractions. Pour cette phase de diffusion, il est essentiel que le patient soit confortablement installé, et que l'on puisse offrir une illusion parfaite : c'est à dire que l'illusion à l'écran soit dans la parfaite continuité de son bras pathologique, afin qu'il soit convaincu que son bras pathologique bouge normalement.

Un autre objectif de l'invention est de fournir un appareil simple et compact. Il est aussi important d'assurer une bonne stabilité d'ensemble.

Afin de résoudre les problèmes précités, il a été mis au point un dispositif pour la mise en oeuvre d'une thérapie miroir, comprenant :
- une caméra pour filmer un membre sain d'un patient,
- une surface de travail sur laquelle le patient positionne son membre sain ou pathologique,
- un écran apte à afficher l'image du membre sain qui est positionné sur la surface de travail et qui est filmé par la caméra, ledit écran empêchant le patient de voir directement son membre sain ou pathologique sur la surface de travail, et
- une structure porteuse qui est connectée à la surface de travail, et sur laquelle l'écran et la caméra sont montés.

Selon invention, le dispositif comprend un miroir fixé derrière l'écran, et la caméra filme le membre sain réfléchi dans le miroir. L'image affichée par l'écran est une réflexion du membre sain dans le miroir.

De cette manière, le dispositif facilite le réglage nécessaire pour mettre en oeuvre la thérapie miroir. L'utilisation de la caméra filmant le membre sain non pas directement, mais par l'intermédiaire du miroir, permet d'adapter le dispositif plus facilement aux besoins de chaque patient.

La surface de travail est conçue pour recevoir de manière confortable le membre sain dans une première phase d'enregistrement, puis le membre pathologique dans une seconde phase de diffusion.

Durant cette phase de diffusion, l'image du membre sain affichée à l'écran se superpose au membre pathologique du patient positionné sur la surface de travail. Le patient peut alors réaliser les exercices de rééducation du membre pathologique.

Selon une caractéristique particulièrement avantageuse, la caméra, l'écran, le miroir et une partie de la structure porteuse sont mobiles par rapport à la surface de travail en formant un système monolithique.

De cette manière, le dispositif est plus simple, et les réglages nécessaires pour sa mise en oeuvre sont minimisés, tout en assurant une grande flexibilité d'adaptation.

De préférence, la structure porteuse est réglable horizontalement par rapport à la surface de travail, en étant rapprochée ou écartée du patient.

Toujours de préférence, la structure porteuse est réglable en hauteur par rapport à la surface de travail, pour faciliter l'installation du membre pathologique du patient (en particulier si ce membre doit être maintenu par une attelle ou un support type mousse).

Selon un mode de réalisation avantageux, une distance constante est définie entre la caméra et le miroir. De préférence, le miroir est fixé parallèlement à l'écran. Ainsi le réglage est encore plus facilité. Il suffit de déplacer la structure porteuse, pour que la distance entre les yeux du patient soit égale à la distance entre la caméra et le miroir. Encore de préférence, un angle, défini entre la surface de travail et un plan coplanaire à l'écran, dans un plan perpendiculaire à la surface de travail et au miroir, a une valeur fixe. Encore plus avantageusement, cet angle est égal à 53°.

Avantageusement, le dispositif comprend un écran de pilotage / contrôle d'une séance de travail, accessible à un praticien.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique du dispositif selon l'invention, montrant le principe d'invention ;
- la figure 2 est une représentation schématique du dispositif en vue isométrique ;
- la figure 3 est une vue de face du dispositif ;
- la figure 4 est une vue de dessus du dispositif ;
- la figure 5 est une vue de côté du dispositif ;
- la figure 6 est une vue de derrière du dispositif ;
- la figure 7 est une vue en coupe du dispositif, selon la ligne VII-VII à la figure 4 ;
- la figure 8 est une vue en perspective du dispositif, montrant les éléments de réglage.

Par soucis de clarté, les mêmes éléments portent les mêmes références numériques sur les différentes figures.

### EXPOSE DETAILLE DE L'INVENTION

La présente invention concerne un dispositif (1) pour la mise en oeuvre d'une thérapie miroir.

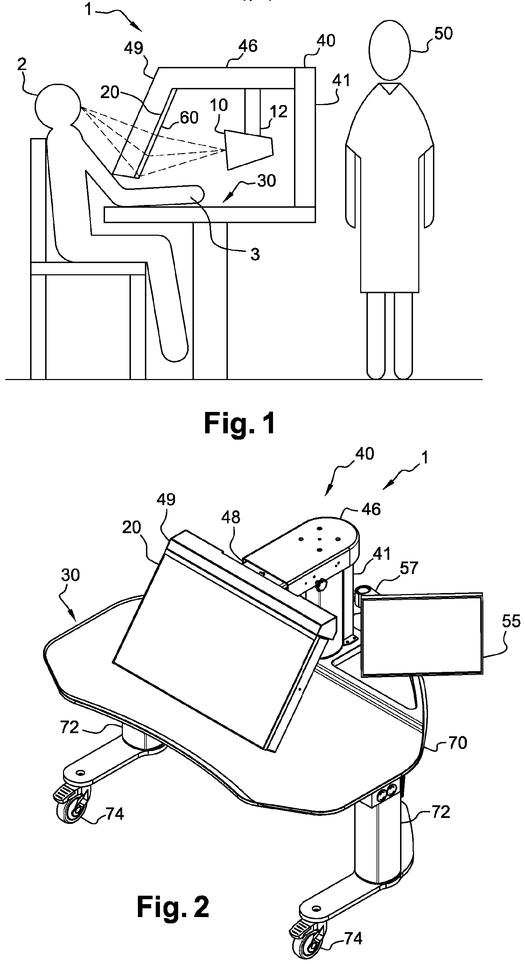

La figure 1 illustre le principe du dispositif (1). De façon connue par l'homme du métier, la thérapie miroir consiste à montrer au patient (2) une image fausse de son membre déficient. En l'espèce, le patient (2) voit une image transformée de son membre sain (3). De ce fait, le cerveau croit que le membre déficient effectue des séquences de mouvements qui, en réalité, sont effectuées par le membre (3) sain.

Grâce à la plasticité du cerveau, la remodélisation de connexions neurales est possible et permet d'obtenir des résultats avantageux pour remédier aux douleurs chroniques, ou à une déficience motrice du membre. La transformation d'image susmentionnée peut être une réflexion dans un miroir, d'où le nom de la thérapie.

Cependant, l'utilisation d'un miroir nécessite que le patient (2) maintienne une position peu confortable pour bien voir la réflexion, ce qui limite fortement le temps des séances thérapeutiques.

Dans la description ci-après, le "membre" désigne le bras et/ou la main du patient (2). En alternative, le "membre" peut être la jambe et/ou le pied du patient (2).

Classiquement, un appareil électronique pour la thérapie miroir comprend une caméra (10) et un écran (20). L'appareil comporte un processeur exécutant un algorithme, qui permet de transformer une image filmée en effectuant une réflexion symétrique et une correction de la distorsion. Cependant, la mise en oeuvre de l'appareil nécessite un réglage minutieux pour chaque patient (2), afin d'obtenir une bonne image, ce que limite toujours le temps disponible pour la thérapie en tant que telle.

Il est nécessaire de remplir les deux conditions suivantes pour que la visualisation soit vraisemblable :
- Le chemin optique total entre l'oeil du patient (2) et son membre sain (3) et le chemin optique entre la caméra (10) et le membre sain (3) du patient (2) doivent être identiques.
- L'angle formé par la ligne visuelle allant des yeux du patient (2) à son membre, ledit angle étant mesuré par rapport à la surface de travail (30), doit être le même que l'angle formé par la ligne visuelle allant de la caméra (10) au miroir (60) mesuré par rapport à la normale au miroir (60).

En résumé, le patient (2) voit son membre à travers l'écran (20) avec la même perspective que s'il le voyait en direct.

Pour répondre à ces exigences de manière sûre, simple et efficace, un dispositif (1), conforme à l'invention, est proposé. Le dispositif (1) comprend, de façon connue :
- une caméra (10) pour filmer un membre sain (3) d'un patient (2) ;
- une surface de travail (30) sur laquelle le patient (2) positionne son membre sain (3) et/ou son membre pathologique ;
- un écran (20) apte à afficher l'image du membre sain (3), qui est positionné sur la surface de travail (30), et qui est filmé par la caméra (10). L'écran (20) empêche le patient (2) de voir directement son membre sain ou déficient sur la surface de travail.

Le dispositif (1) comprend également une structure porteuse (40), qui est connectée à la surface de travail (30), et sur laquelle l'écran (20) et la caméra (10) sont montés. La structure porteuse (40) sera détaillée ci-après.

Le dispositif (1) est apte à être utilisé par le patient (2) en autonomie, ou sous la surveillance d'un praticien (50) qui peut être un docteur, un kinésithérapeute, ou un autre professionnel de santé. Le fonctionnement en autonomie est notamment rendu possible par un logiciel comprenant des descriptions d'exercices. Le praticien (50) dispose d'un autre logiciel dédié lui permettant, en outre, d'évaluer l'efficacité de la thérapie, et de planifier le traitement.

Selon l'invention, un miroir (60) est fixé derrière l'écran (20). Or, la caméra (10) filme le membre sain (3) réfléchi dans le miroir (60). Autrement dit, l'image affichée par l'écran (20) est une réflexion du membre (3) dans ledit miroir (60). Le miroir (60) est fixé parallèlement à l'écran (20), pour assurer une égalité des angles déjà mentionnés.

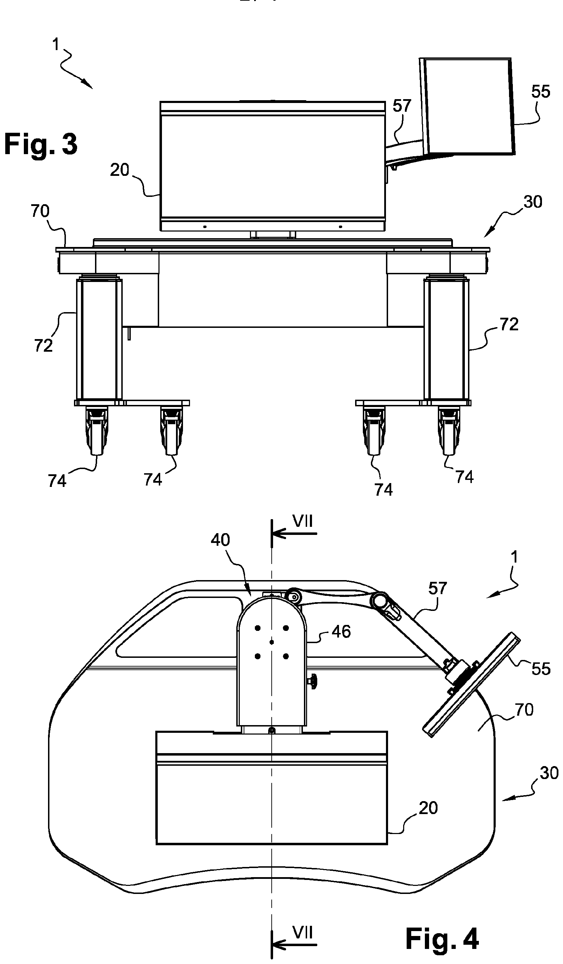

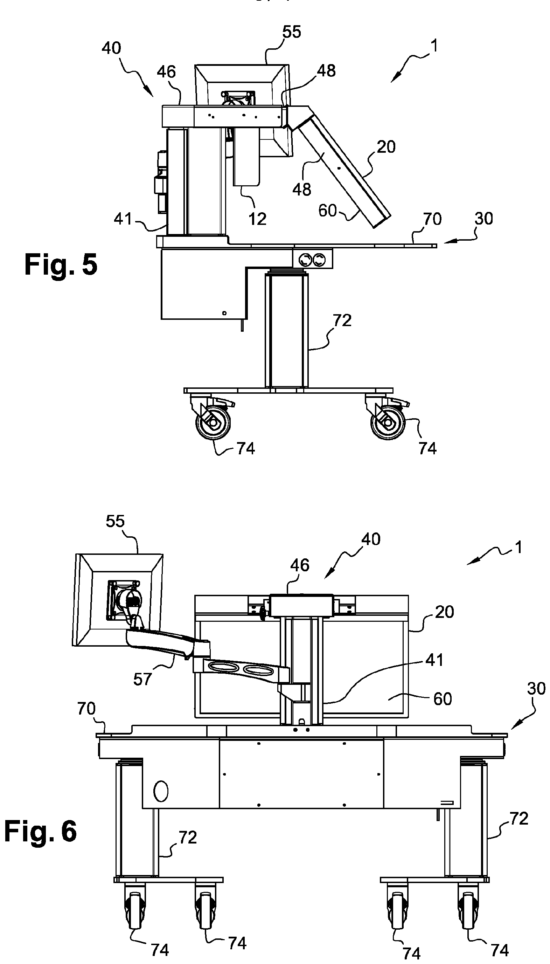

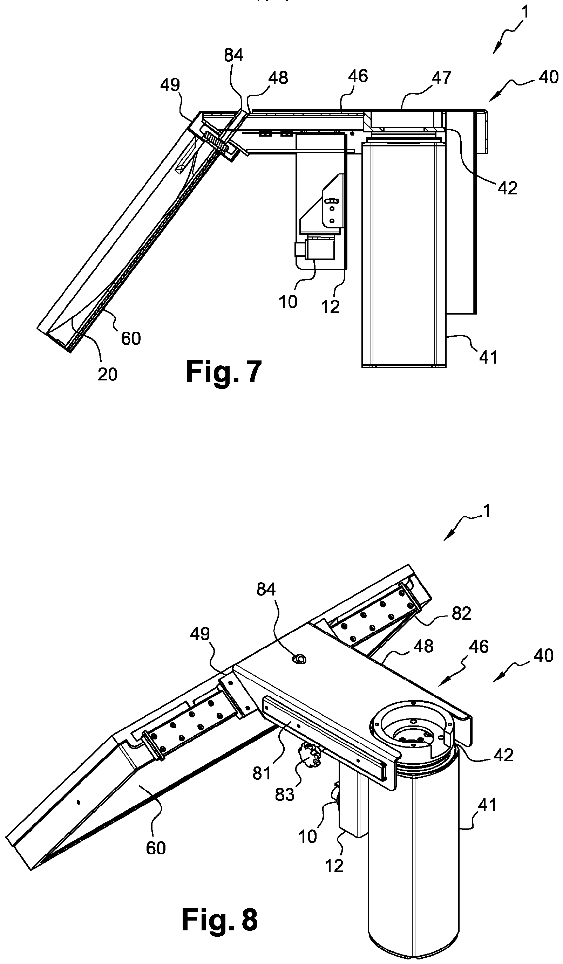

En référence aux figures 2 à 8, les détails de construction du dispositif (1) seront maintenant décrits.

La surface de travail (30) est de préférence une table (70). La table (70) est installée sur des pieds (72) réglables en hauteur, avec des roulettes (74) sur leurs extrémités pour faciliter le déplacement et le stockage du dispositif (1). La surface de travail (30) est filmée par la caméra (10). Selon les expérimentations ayant abouti à la conception du dispositif (1), la taille optimale de la surface de travail (30) est de l'ordre de 800 mm de largeur dans la direction droite-gauche pour le patient (2) utilisant le dispositif (1), et 450 mm de longueur dans la direction de l'axe entre le patient (2) et la caméra (10).

La présente description concerne plus particulièrement un dispositif (1) adapté à la thérapie des membres supérieurs. Bien entendu, le dispositif (1) peut aussi bien être utilisé pour la thérapie miroir d'un membre inférieur. Dans ce cas, la surface de travail (30) est disposée plus bas, et sa taille est adaptée en conséquence. Dans cette variante de réalisation, le principe de l'invention reste le même, de sorte que les différences structurelles ne seront pas décrites en détail.

La structure porteuse (40) comprend deux parties. Une partie fixe (41) est solidaire de la table (70). Une partie mobile (46) est articulée par rapport à la partie fixe (41). La caméra (10), l'écran (20), le miroir (60) et la partie mobile (46) forment un système monolithique. La partie mobile (46) comprend deux éléments horizontaux, un guide (47) et un coulisseau (48) et, et un élément incliné (49). La caméra (10) est disposée dans un support (12) de caméra (10), qui est solidaire du coulisseau (48) de la partie mobile (46). L'écran (20) et le miroir (60) sont solidaires de l'élément incliné (49) de la partie mobile (46).

La structure porteuse (40) est réglable horizontalement par rapport à la surface de travail (30), la partie mobile (46) étant rapprochée ou écartée du patient (2). Ce réglage est possible, par exemple grâce à une glissière (81), permettant une translation entre le guide (47) et le coulisseau (48) de la partie mobile (46).

Avantageusement, l'écran (20) est aussi réglable latéralement. Ce réglage est possible, par exemple grâce à une glissière (82), permettant une translation entre l'écran (20), solidaire de l'élément inclinée (49), et le coulisseau (48).

La structure porteuse (40) est également réglable en hauteur par rapport à la surface de travail (30). A cet effet, la partie fixe (41) intègre un pied télescopique (42) permettant de modifier la hauteur de la partie mobile (46) par rapport à ladite table (30). Le pied (42) est fixé au guide (47), par exemple par des vis.

Le thérapeute (50) peut choisir de monter ou descendre la partie mobile (46) et donc l'écran (20) en fonction du patient (2) (ceci afin d'installer confortablement le patient (2), en particulier son bras pathologique avec attelle ou support par exemple).

Autrement dit, le guide (47) de la partie mobile (46) est déplaçable en hauteur par rapport à la surface de travail (30). Par conséquent, le coulisseau (48) est aussi déplaçable en hauteur, et est en plus mobile en translation selon une direction horizontale par rapport au guide (47). Finalement, l'élément incliné (49) est mobile selon une direction verticale et selon deux directions horizontales.

Il est important de mentionner que le réglage latéral de l'écran (20) selon l'axe de la glissière (82) permet de positionner l'image d'un membre à l'écran (20) dans la continuité du membre pathologique du patient (2), et ce afin de favoriser l'illusion. Le patient (2) croit ainsi que c'est son vrai membre à l'écran (20).

Les glissières (81) et (82) sont dotées de molettes, respectivement (83) et (84), pour bloquer la position du coulisseau (47) et de l'élément (48) une fois leur réglage effectué.

Pour utiliser l'invention, le patient (2) s'installe sur une chaise devant le dispositif (1). Une séance se déroule en deux phases : une première phase d'enregistrement et une seconde phase de diffusion.

La première phase comprend les étapes suivantes :
- Le patient (2) pose son membre sain (3) sur la surface de travail (30).
- Des réglages sont effectués pour assurer une position confortable du patient (2) et pour permettre à la caméra (10) d'enregistrer correctement une réflexion du membre sain (3).
- Une vidéo de mouvements effectués par le membre sain (3) est enregistrée.
- Plusieurs mouvements peuvent être effectués, et chacun est enregistré séparément.
- Le patient (2) retire son membre sain (3) de la surface de travail (30).

Ensuite, le système inverse les vidéos, de sorte que, par exemple, un membre droit est apte à être affiché comme un membre gauche.

La deuxième phase comprend les étapes suivantes :
- Le patient (2) pose son membre pathologique sur la surface de travail (30).
- Des réglages sont effectués pour assurer une position confortable du patient (2) et pour que le membre à l'écran (20) soit affiché dans la continuité du membre pathologique, c'est-à-dire en superposant l'image modifiée du membre sain (3) avec la localisation du membre pathologique.
- L'affichage du film inversé est lancé sur l'écran (20).

Pour expliquer plus en détail les réglages de la première phase, le dispositif (1) est considéré comme correctement réglé lorsque le chemin optique total entre l'oeil du patient (2) et son membre sain (3) est égal au chemin optique entre la caméra (10) et le membre sain (3) du patient (2), c'est-à-dire la distance entre la caméra (10) et le miroir (60) plus la distance entre le miroir (60) et le membre sain (3) du patient (2).

Lorsque la structure porteuse (40) est ajustée, le membre sain (3), réfléchi dans le miroir (60), est filmé par la caméra (10) avec une relation géométrique identique à celle avec laquelle le patient (2) regarde l'image sur l'écran (20).

Avantageusement, le praticien (50) dispose d'un écran (55) dédié, installé sur un bras (57) permettant de changer facilement sa position. Cet écran (55) permet au praticien (50) de piloter / contrôler la séance de travail du patient (2).

Ainsi, comme cela ressort de la description qui précède, le dispositif (1) selon l'invention est facile à mettre en oeuvre, avec notamment, une capacité de réglage commode. Il est à noter que la zone de travail est toujours bien adaptée pour générer une image plausible afin d'obtenir un effet d'illusion nécessaire à la mise en oeuvre de la thérapie.

## Revendications

1. Dispositif (1) pour la mise en oeuvre d'une thérapie miroir (60), comprenant :
- une caméra (10) pour filmer un membre sain (3) d'un patient (2),
- une surface de travail (30) sur laquelle le patient (2) positionne son membre sain (3) ou pathologique,
- un écran (20) apte à afficher l'image du membre sain (3), qui est positionné sur la surface de travail (30), et qui est filmé par la caméra (10), ledit écran (20) empêchant le patient (2) de voir directement son membre sain (3) ou pathologique sur la surface de travail (30), et
- une structure porteuse (40), qui est connectée à la surface de travail (30), et sur laquelle l'écran (20) et la caméra (10) sont montés,
***caractérisé* en ce que** le dispositif (1) comprend un miroir (60) fixé derrière l'écran *(20), et **en ce que*** la caméra (10) filme le membre sain (3) réfléchi dans le miroir (60), ***et en ce que*** l'image affichée par l'écran (20) est une réflexion du membre sain (3) dans ledit miroir (60).

2. Dispositif (1) selon la revendication 1, ***caractérisé* en ce que** la caméra (10), l'écran (20), le miroir (60) et une partie (46) de la structure porteuse (40) sont mobiles par rapport à la surface de travail (30) en formant un système monolithique.

3. Dispositif (1) selon l'une quelconque des revendications précédentes, ***caractérisé* en ce que** la structure porteuse (40) est réglable horizontalement par rapport à la surface de travail (30), en étant rapprochée ou écartée du patient (2).

4. Dispositif (1) selon l'une quelconque des revendications précédentes, ***caractérisé* en ce que** la structure porteuse (40) est réglable en hauteur par rapport à la surface de travail (30).

5. Dispositif (1) selon l'une quelconque des revendications précédentes, ***caractérisé* en ce qu'**une distance constante est définie entre la caméra (10) et le miroir (60).

6. Dispositif (1) selon l'une quelconque des revendications précédentes, ***caractérisé* en ce que** le miroir (60) est fixé parallèlement à l'écran (20).

7. Dispositif (1) selon l'une quelconque des revendications précédentes, ***caractérisé* en ce qu'**un angle défini entre la surface de travail (30) et un plan coplanaire à l'écran (20), dans un plan perpendiculaire à la surface de travail (30) et au miroir (60), a une valeur fixe.

8. Dispositif (1) selon la revendication 7, ***caractérisé* en ce que** l'angle est égal à 53°.

9. Dispositif (1) selon l'une quelconque des revendications précédentes, ***caractérisé* en ce qu'**il comprend un écran (55) de pilotage / contrôle d'une séance de travail, accessible à un praticien (50).

## Patentansprüche

1. Vorrichtung (1) zur Durchführung einer Spiegeltherapie (60), umfassend:
- eine Kamera (10) zum Filmen eines gesunden Gliedmaßes (3) eines Patienten (2),
- eine Arbeitsfläche (30), auf der der Patient (2) sein gesundes (3) oder pathologisches Gliedmaß positioniert,
- einen Bildschirm (20), der geeignet ist, das Bild des gesunden Gliedmaßes (3) anzuzeigen, das auf der Arbeitsfläche (30) positioniert und von der Kamera (10) gefilmt wird, wobei der genannte Bildschirm (20) verhindert, dass der Patient (2) sein gesundes (3) oder pathologisches Gliedmaß direkt auf der Arbeitsfläche (30) sieht, und
- eine Tragstruktur (40), die mit der Arbeitsfläche (30) verbunden ist und auf der der Bildschirm (20) und die Kamera (10) montiert sind, **dadurch gekennzeichnet, dass** die Vorrichtung (1) einen Spiegel (60) aufweist, der hinter dem Bildschirm (20) befestigt ist, und dass die Kamera (10) das gesunde Gliedmaß (3) filmt, das im Spiegel (60) reflektiert wird, und dass das vom Bildschirm (20) angezeigte Bild eine Reflexion des gesunden Gliedmaßes (3) in dem genannten Spiegel (60) ist.

2. Vorrichtung (1) nach Anspruch 1, **dadurch gekennzeichnet, dass** die Kamera (10), der Bildschirm (20), der Spiegel (60) und ein Teil (46) der Tragstruktur (40) in Bezug auf die Arbeitsfläche (30) beweglich sind und ein monolithisches System bilden.

3. Vorrichtung (1) nach einem der vorhergehenden Ansprüche, **dadurch gekennzeichnet, dass** die Tragstruktur (40) horizontal in Bezug auf die Arbeitsfläche (30) verstellbar ist, indem sie dem Patienten (2) angenähert oder von ihm entfernt wird.

4. Vorrichtung (1) nach einem der vorhergehenden Ansprüche, **dadurch gekennzeichnet, dass** die Tragstruktur (40) in der Höhe in Bezug auf die Arbeitsfläche (30) verstellbar ist.

5. Vorrichtung (1) nach einem der vorhergehenden Ansprüche, **dadurch gekennzeichnet, dass** ein konstanter Abstand zwischen der Kamera (10) und dem Spiegel (60) definiert ist.

6. Vorrichtung (1) nach einem der vorhergehenden Ansprüche, **dadurch gekennzeichnet, dass** der Spiegel (60) parallel zum Bildschirm (20) befestigt ist.

7. Vorrichtung (1) nach einem der vorhergehenden Ansprüche, **dadurch gekennzeichnet, dass** ein definierter Winkel zwischen der Arbeitsfläche (30) und einer zur Bildschirmfläche (20) koplanaren Ebene, in einer zur Arbeitsfläche (30) und zum Spiegel (60) senkrechten Ebene, einen festen Wert hat.

8. Vorrichtung (1) nach Anspruch 7, **dadurch gekennzeichnet, dass** der Winkel gleich 53° ist.

9. Vorrichtung (1) nach einem der vorhergehenden Ansprüche, **dadurch gekennzeichnet, dass** sie einen Steuerungs-/Kontrollbildschirm (55) für eine Arbeitssitzung umfasst, der für einen Praktiker (50) zugänglich ist.

## Claims

1. Device (1) for implementing a mirror therapy (60), comprising:
- a camera (10) for filming a healthy limb (3) of a patient (2),
- a working surface (30) on which the patient (2) positions their healthy (3) or pathological limb,
- a screen (20) capable of displaying the image of the healthy limb (3), which is positioned on the working surface (30), and which is filmed by the camera (10), said screen (20) preventing the patient (2) from directly seeing their healthy (3) or pathological limb on the working surface (30), and
- a support structure (40), which is connected to the working surface (30), and on which the screen (20) and the camera (10) are mounted, **characterized in that** the device (1) includes a mirror (60) fixed behind the screen (20), and **in that** the camera (10) films the healthy limb (3) reflected in the mirror (60), and **in that** the image displayed by the screen (20) is a reflection of the healthy limb (3) in said mirror (60).

2. Device (1) according to claim 1, **characterized in that** the camera (10), screen (20), mirror (60), and a part (46) of the support structure (40) are movable with respect to the working surface (30), forming a monolithic system.

3. Device (1) according to any one of the preceding claims, **characterized in that** the support structure (40) is horizontally adjustable with respect to the working surface (30), being brought closer to or moved away from the patient (2).

4. Device (1) according to any one of the preceding claims, **characterized in that** the support structure (40) is height-adjustable with respect to the working surface (30).

5. Device (1) according to any one of the preceding claims, **characterized in that** a constant distance is defined between the camera (10) and the mirror (60).

6. Device (1) according to any one of the preceding claims, **characterized in that** the mirror (60) is fixed parallel to the screen (20).

7. Device (1) according to any one of the preceding claims, **characterized in that** a defined angle between the working surface (30) and a plane coplanar with the screen (20), in a plane perpendicular to the working surface (30) and the mirror (60), has a fixed value.

8. Device (1) according to claim 7, **characterized in that** the angle is equal to 53°.

9. Device (1) according to any one of the preceding claims, **characterized in that** it includes a control/display screen (55) for a work session, accessible to a practitioner (50).
